# EUROPEAN PATENT APPLICATION

(11) **EP 1 908 758 A1**
(43) Date of publication of application: **09.04.2008**
(21) Application number: 06380259.9
(22) Date of filing: 04.10.2006
(51) Int. Cl.: C07D 313/12, C07C 229/34

(54) **Process for the preparation of (Z)-[11-(3-dimethylamino-propyliden)-6,11-dihydro-dibenzo[b,e]oxepin-2-yl]-acetic acid hydrochloride**

(71) Applicant: Urquima S.A., 08105 Sant Fost de Campsentelles, Barcelona (ES)
(72) Inventor: Bachs Roca, Jorge, 08021 Barcelona (ES); Pujol Ollé, Xavier, 08025 Barcelona (ES)
(74) Representative: Isern-Jara, Nuria

(57) **Abstract**

The present invention relates an optimised process for the preparation of the compound of formula (Z)-[11-(3-dimethylamino-propyliden)-6,11-dihydro-dibenzo[*b,e*]oxepin-2-yl]-acetic acid hydrochloride in a higher purity, which comprises the preparation of salts of advanced intermediates and final crystallization of compound of formula 11-[(Z)-3-(dimethylamino)propylidene]-6,11-dihydro-dibenz[*b,e*]oxepin-2-yl]-acetic acid hydrochloride.

## Description

### Field of the invention

The present invention provides an optimized process for the preparation of (Z)-[11-(3-dimethylamino-propyliden)-6,11-dihydro-dibenzo[*b*,e]oxepin-2-yl]-acetic acid hydrochloride and its intermediates of the synthesis in a higher purity.

### Description of the prior art

The compound 11-[(Z)-3-(dimethylamino)propylidene]-6,11-dihydrodibenz[*b,e*]oxepin-2-yl]-acetic acid represented by formula (II), commonly known as Olopatadine, has been used as an active constituent drug, in form of its hydrochloric salt.

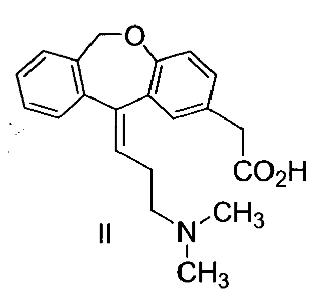

During the synthesis of (Z)-olopatadine Hydrochloride a number of impurities are formed, of which (E) isomer of olopatadine is one of the most significant single impurity. In order to have a marketing approval of a drug, the regulatory authority requires that the drug is free from impurities.

The preparation of the compound of formula (II) has been described previously in patents number US5115883 and US4871865. While olopatadine base (II) is purified by column chromatography in US5115883, olopatadine base of formula II is crystallised from water in US4871865.

Journal of Medicinal Chemistry, 1992, vol.35, no11, p2083 described the preparation of olopatadine hydrochloride of formula (II) by separation of (E)-olopatadine of formula (II) from a mixture of isomers of a tosylate salt of olopatadine base of formula (II) followed by preparation of its corresponding hydrochloride salt.

A new process of preparation of olopatadine hydrochloride of formula (I) was claimed in patent application WO2006010459, in which olopatadine hydrochloride is isomerically pure prepared.

Due to the difficulty of obtaining an olopatadine hydrochloride of formula (I) with a high purity which imply a separation of the main important impurity, its (E)-isomer, it is necessary a new process for the preparation of pure olopatadine hydrochloride of formula I which is suitable for its preparation at industrial scale.

### Summary of the invention

The present invention relates an optimised process for the preparation of the compound of formula (I) in a higher purity, based on the process of preparation claimed in patent application WO00601049, which comprises the preparation of salts of advanced intermediates and final crystallization of compound of formula (I).

The higher purity of intermediates are obtained by preparation of its corresponding organic or inorganic salts, which imply a higher purity of compound of formula (I), which is purified by crystallization to obtain the required purity by the regulatory authorities.

One aspect of the present invention relates a process for the preparation of compound of formula (I), 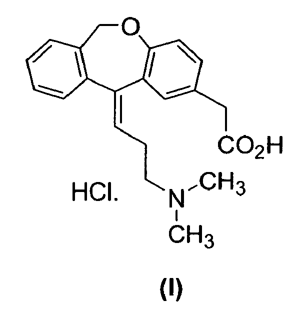 which comprises the reaction of compound of formula (III) 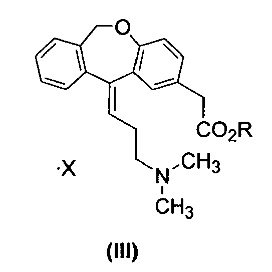 with a base, wherein R is C1-4 alkyl and X is an acid addition salt, to obtain compound of formula (II), 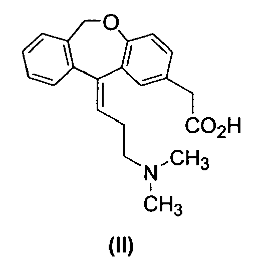 and reacting compound of formula (II) with hydrochloric acid, to obtain compound of formula (I), crystallizing with tetrahydrofurane and crystallizing with a ketone and water.

Another aspect of the present invention comprises a process of preparation of compound of formula (III) reacting compound of formula (IV), 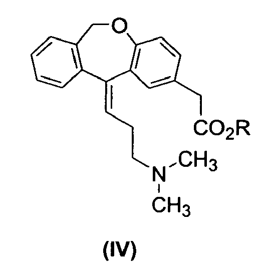 with an acid in one or more solvents or mixtures thereof, wherein R is C1-4 alkyl and X is an acid addition salt.

Another aspect of the present invention comprises a process for the preparation of compound of formula (IV) reacting compound (V), 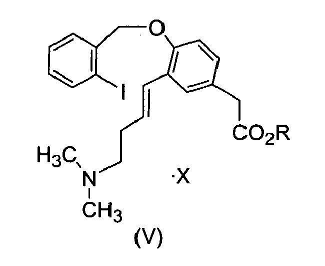 with a palladium catalyst, wherein R is C1-4 alkyl and X is an acid addition salt.

Another aspect of the present invention comprises a process for the preparation of compound of formula (V) reacting compound (VI), 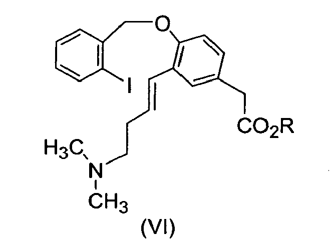 with an acid in one or more solvents or mixtures thereof, wherein R is C1-4 alkyl and X is an acid addition salt.

Another aspect of the present invention comprises a compound of formula (V), 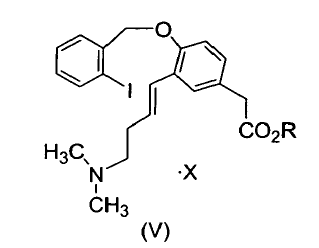 wherein R is C1-4 alkyl and X is an acid addition salt.

Another aspect of the present invention comprises a compound of formula (III), 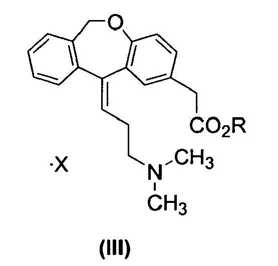 wherein R is C1-4 alkyl and X is an acid addition salt.

Another aspect of the present invention relates a process for the preparation of compound of formula (III), 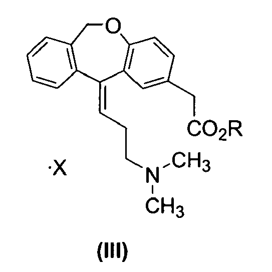 which comprises reacting compound (IV), 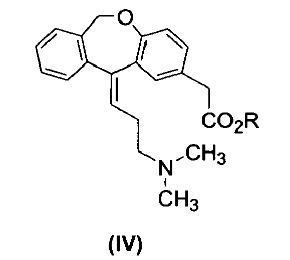 with an acid in one or more solvents or mixtures thereof, wherein R is C1-4 alkyl and X is an acid addition salt.

### Description of the invention

Within the definitions that are mentioned, the term C1-4 alkyl means a linear or branched chain of 1 to 4 carbon atoms, for example methyl, ethyl, propyl, isopropyl, butyl, isobutyl and tert-butyl.

X is the acid used to form the addition salt, examples of acids that could be used are hydrochloric acid, hydrobromic acid, succinic acid, oxalic acid, fumaric acid maleic acid or sulfuric acid.

The meaning of a ketone is a crystallising solvent with a ketone moiety of formula R1-CO-R2, where R1 and R2 are C1-4 alkyl. For example acetone, methylisobutylketone, methylethylketone, diisobutylketone or mixtures thereof.

One aspect of the present invention relates a process of preparation of compound of formula (I) which comprises the reaction of compound of formula III with a base, wherein R is C1-4 alkyl and X is an acid addition salt, to obtain compound of formula (ii), and reacting compound of formula (ii) with hydrochloric acid, to obtain compound of formula (I), crystallizing with tetrahydrofurane and crystallizing with a ketone and water.
In a preferred embodiment base is sodium hydroxide.
In another preferred embodiment R is methyl.
In another preferred embodiment X is selected from hydrochloric acid, hydrobromide acid, succinic acid, oxalic acid, fumaric acid, maleic acid or sulfuric acid, more preferred embodiment is oxalic acid.
In another preferred embodiment ketone is selected from acetone, methylisobutylketone, methylethylketone, diisobutylketone or mixtures thereof, more preferred embodiment is acetone.
In a more preferred embodiment the base is sodium hydroxide, R is methyl, X is oxalic acid and ketone is acetone.

Another aspect of the present invention comprises a process for the preparation of compound (III) reacting compound of formula (IV), with an acid in one or more solvents or mixtures thereof, wherein R is C1-4 alkyl and X is as defined above.
In a preferred embodiment acid is hydrochloric acid, hydrobromic acid, succinic acid, oxalic acid, fumaric acid, maleic acid or sulfuric acid. In a more preferred embodiment acid is oxalic acid. In a more preferred embodiment acid is oxalic acid dihydrate.
In another preferred embodiment solvents are one or more alcohols, one or more esters or mixtures thereof. In a more preferred embodiment solvents are ethanol, ethyl acetate and mixtures thereof.
In another preferred embodiment R is methyl.
In a more preferred embodiment acid is oxalic acid dihydrate, solvents are ethanol, ethyl acetate or mixtures thereof and R is methyl.

Another aspect of the present invention comprises a process for the preparation of compound of formula (IV) reacting compound (V), with a palladium catalyst, wherein R is C1-4 alkyl and X is as defined above.
In a preferred embodiment R is methyl.
In another preferred embodiment a palladium catalyst is a fibre-anchored palladium homogeneous catalysts. In a more preferred embodiment a palladium catalyst is Fibrecat 1001® (Johnson Matthey).
In a more preferred embodiment R is methyl and a palladium catalyst is Fibrecat 1001® (Johnson Matthey).

Another aspect of the present invention comprises a process for the preparation of compound of formula (V) reacting compound (VI), with an acid in one or more solvents or mixtures thereof, wherein R is C1-4 alkyl and X is as defined above.
In a preferred embodiment R is methyl.
In another preferred embodiment acid comprises hydrochloric acid, hydrobromic acid, succinic acid, oxalic acid, fumaric acid, maleic acid or sulfuric acid. In a more preferred embodiment acid comprises oxalic acid and in a more preferred embodiment acid comprises oxalic acid dihydrate.
In another preferred embodiment solvents comprise one or more alcohols, one or more esters or mixtures thereof, in a more preferred embodiment solvents comprise ethanol, ethyl acetate or mixtures thereof.
In a more preferred embodiment R is methyl, acid is oxalic acid dihydrate and solvents are ethanol, ethyl acetate or mixtures thereof.

Another aspect of the invention comprises a compound of formula (V) wherein R is C1-4 alkyl and X is an acid addition salt.
In a preferred embodiment of compound of formula (V), R is methyl.
In another preferred embodiment of compound of formula (V), X comprises hydrochloric acid, hydrobromic acid, succinic acid, oxalic acid, fumaric acid, maleic acid or sulfuric acid. In a more preferred embodiment X is oxalic acid.
In a more preferred embodiment of compound of formula (V), R is methyl and X is oxalic acid.
In another more preferred embodiment of compound of formula (V), (V) comprises (Z)-{4-[2-(4-dimethylamino-but-1-enyl)-benzyloxy]-3-iodo-phenyl}-acetic acid methyl ester oxalate.

Another aspect of the invention comprises a compound of formula (III) wherein R is C1-4 aikyi and X is an acid addition salt.
In a preferred embodiment of compound of formula (III), R is methyl.
In another preferred embodiment of compound of formula (III), X comprises hydrochloric acid, hydrobromic acid, succinic acid, oxalic acid, fumaric acid, maleic acid or sulfuric acid. In a more preferred embodiment X is oxalic acid.
In a more preferred embodiment of compound of formula (III), R is methyl and X is oxalic acid.

In another preferred embodiment of compound of formula (III), (III) comprises (Z)-[11-(3-dimethylamino-propilidene)-6,11-dihydro-dibenzo[*b*,e]oxepin-2-yl]acetic acid methyl ester oxalate.

Another aspect of the present invention relates the preparation of compound of formula (III), which comprises reacting compound (IV), with an acid in one or more solvents or mixtures thereof, wherein R is C1-4 alkyl and X is an acid addition salt.
In a preferred embodiment R is methyl.
In another preferred embodiment acid comprises hydrochloric acid, hydrobromic acid, succinic acid, oxalic acid, fumaric acid, maleic acid or sulfuric acid. In a more preferred embodiment acid is oxalic acid and in a more preferred embodiment acid is oxalic acid dihydrate.
In another preferred embodiment X comprises hydrochloric acid, hydrobromic acid, succinic acid, oxalic acid, fumaric acid, maleic acid or sulfuric acid. In a more preferred embodiment X is oxalic acid.
In another preferred embodiment solvents comprise one or more alcohols, one or more esters or mixtures thereof. In a more preferred embodiment solvents comprise ethanol, ethyl acetate or mixtures thereof.
In a more preferred embodiment R is methyl, acid is oxalic acid dihydrate, X is oxalic acid and solvents are ethanol, ethyl acetate or mixtures thereof.

Starting compounds of formula (VI) can be prepared by methods described in the literature, such as patent application number WO2006010459.

The invention, which is illustrated by the following examples, is not to be understood as being limited in any way.

### Example 1. (E)-[3-(4-dimethy)amino-but-1-enyl)-4-(2-iodo-benzyloxy)-phenyl]-acetic acid methyl ester

Lithium bis(trimethylsilyl)amide (LiHMDS) (1M·THF, 51.5mL) was slowly added to a solution of (3-dimethylamino-propyl)-triphenylphosphonium iodide (24.33g; 51.18mmol) in anhydrous toluene (225mL) with mechanical stirring and inert atmosphere. The mixture was stirred at room temperature (20-25°C) for 1h. Following this, a solution of [3-formyl-4-(2-iodo-benzyloxy)-phenyl]-acetic acid methyl ester (5g, 12.19mmol) in anhydrous toluene (127mL) was slowly added to the mixture and they were stirred at room temperature for 2h.

Water (150mL) was added to the mixture at 25°C and the solid obtained was filtered and dried. Once the filtrate and layers had separated, the aqueous layer was washed with toluene (67mL). Organic layers were collected and washed with HCl 2N (x2). Acidic layers were collected, basified with anhydrous solid Na₂CO₃ and extracted with ethyl acetate. Organic layers were washed with water, and dried with anhydrous Na₂SO₄, filtered and concentrated to dryness under reduced pressure to obtain 5.5g [3-(4-dimethylamino-but-1-enyl)-4-(2-iodo-benzyloxy)-phenyl]-acetic acid methyl ester (92.86% chromatographic purity; *E*/*Z*: 89.9:10.2)

### Example 2. (E)-[3-(4-dimethylamino-but-1-enyl)-4-(2-iodo-benzyloxy)-phenyl]-acetic acid methyl ester oxalate

A solution of (*E*)-[3-(4-dimethylamino-but-1-enyl)-4-(2-iodo-benzyloxy)-phenyl]-acetic acid methyl ester (5.5g; 11.47mmol) in ethyl acetate (35mL) was added to a solution of oxalic acid dihydrate (1.6g; 12.69mmol) in ethanol (10mL). The mixture was stirred at room temperature for 2h. The obtained solid was filtered, washed with ethyl acetate and was dried at 50°C to obtain 5.5g of [3-(4-dimethylamino-but-1-enyl)-4-(2-iodo-benzyloxy)-phenyl]-acetic acid methyl ester oxalate (94.68% chromatographic purity; *E*/*Z*: 90.1:9.1)
¹H-RMN (300 MHz, DMSO-d₆), δ (ppm): 2,46 (m, 2H, -CH₂-CH=), 2,71 [s, 6H, -N(CH₃)₂], 3,08 (m, 2H, -N-CH₂-CH₂-), 3,59 (s, 5H, -CO₂CH₃ and Ar-CH₂-CO₂CH₃), 5,26 (broad signal, 2H, Ar-CH₂-O-), 6,10 (m, 1H, -CH₂-CH=CH-), 6,75 (d, *J* = 15,9 Hz, 1H, -CH₂-CH=CH-), 6,97 (d, 1H, Ar-H), 7,06 (m, 2H, Ar-H), 7,40 (m, 2H, Ar-H), 7,50 (d, 1H, Ar-H) and 7,90 (d, 1H, Ar-H).

### Example 3. (Z)-[11-(3-dimethylamino-propilidene)-6,11-dihydro-dibenzo[b,e]oxepin-2-yl]acetic acid methyl ester

In a reactor with a mechanical stirring and inert atmosphere, were mixed (*E*)-[3-(4-dimethylamino-but-1-enyl)-4-(2-iodo-benzyloxy)-phenyl]-acetic acid methyl ester oxalate (10.45g; 18.35mmol), K₂CO₃ (6,34g; 45,9mmol), tetrabutylammonium bromide (2,96 g; 9,18 mmol) in a mixture of acetonitrile:water (10:1). The mixture was stirred about 15min at room temperature and Fibrecat ® 1001 (3.87g; 1.83mmol) was added. The mixture was heated at 72-74°C for 24h. Following this, the mixture was collected at room temperature, filtered and washed with acetonitrile (50mL) and the filtrate was concentrated to dryness. The obtained solid, was slurred in ethyl acetate and filtered, the resultant filtrate was washed with saturated solution of NaHCO3 and NaCl solution. After that, active carbon (Norit SX Ultra) and diatomaceous earth were added; the slurry was stirred for 30min, filtered and washed with ethyl acetate. The filtrate was dried with anhydrous Na₂SO₄, filtered, washed with ethyl acetate (30mL) and concentrated to dryness under reduced pressure at 55°C to obtain 5,5 g of (*Z*)-[11-(3-dimethylamino-propilidene)-6,11-dihydro-dibenzo[*b*,*e*]oxepin-2-yl]acetic acid methyl ester (90.95% chromatographic purity; *Z*/*E*: 92.6:7.4)

### Example 4. (Z)-[11-(3-dimethylamino-propilidene)-6,11-dihydro-dibenzo[b,e]oxepin-2-yl]acetic acid methyl ester oxalate

A solution of (*Z*)-[11-(3-dimethylamino-propilidene)-6,11-dihydro-dibenzo[*b*,*e*]oxepin-2-yl]acetic acid methyl ester (5.5g; 15.65mmol) in ethyl acetate (34mL) was added to a solution of oxalic acid dihydrate (2.2g; 6.25mmol) in ethanol (13mL), the mixture was stirred and heated at 33-37°C for 1h. Following this, the mixture was cooled at 10-15°C for 1h. The resultant solid was filtered, washed with ethyl acetate and after that with ethanol, the solid was dried at 50°C to obtain 5.5g of (*Z*)-11-(3-dimethylamino-propilidene)-6,11-dihydro-dibenzo[*b*,*e*]oxepin-2-yl]acetic acid methyl ester oxalate (96.57% chromatographic purity; *Z*/*E*: 92.3:7.7)
¹H-RMN (300 MHz, DMSO-d₆), δ (ppm): 2,66 [s, 6H, -N(CH₃)₂], 2,70 (m, 2H, -CH₂-CH=), 3,16 (m, 2H, -N-CH₂-CH₂-), 3,56 (s, 3H, -CO₂CH₃), 3,59 (s, 2H, ArCH₂-CO₂CH₃), 5,14 (broad signal, 2H, Ar-CH₂-O-), 5,60 (t, *J* = 7,2 Hz, 1H, -CH₂-CH=C-), 6,75 (d, 1H, Ar-H), 7,22 (m, 2H, Ar-H), 7,25 (t, 1H, Ar-H), and 7,34 (m, 3H, Ar-H).

### Example 5. (Z)-[11-(3-dimethylamino-propilidene)-6,11-dihydro-dibenzo[b,e]oxepin-2-yl]acetic acid methyl esther hydrochloride

A solution of hydrochloride in ethanol was added to a solution of (*Z*)-[11-(3-dimethylamino-propilidene)-6,11-dihydro-dibenzo[*b*,*e*]oxepin-2-yl]acetic acid methyl esther (93.26% chromatographic purity, 8.2/85.03. *E*/*Z*) in toluene until pH=1. The reaction mixture was concentrated and diethyl ether was added. The mixture was concentrated to dryness and dried to obtain (*Z*)-[11-(3-dimethylamino-propilidene)-6,11-dihydro-dibenzo[*b*,*e*]oxepin-2-yl]acetic acid methyl esther hydrochloride (90.94% chromatographic purity; 7.93/83.01, *E*/*Z).*

### Example 6. (Z)-[11-(3-dimethylamino-propyliden)-6,11-dihydro-dibenzo[b,e]oxepin-2-yl]-acetic acid

NaOH 5N (22.4mL) was added to a slurry of (*Z*)-[11-(3-dimethylamino-propilidene)-6,11-dihydro-dibenzo[*b*,*e*]oxepin-2-yl]acetic acid methyl ester oxalate (15g; 33.95mmol) in water (150mL) at room temperature. The mixture was heated at reflux temperature for 45min, after that, the mixture was cooled at room temperature and HCl 2N was added to adjust the pH between 8,0-8,5. Active carbon (Norit SX Ultra) was added, filtered and washed with water (10mL). The mixture was neutralised with HCl 2N, the solution was seeded with base olopatadine and crystallised. The slurry was cooled at 18-22°C and was stirred for 12-14h. The mixture was centrifuged and dried at 65°C, the resultant solid was washed with water at 40-45°C, after that, was cooled, centrifuged and rewashed with water, dried at 65°C to obtain 7.5g of (*Z*)-[11-(3-dimethylamino-propyliden)-6,11-dihydro-dibenzo[*b*,*e*]oxepin-2-yl]-acetic acid (99.21% chromatophafic purity; *Z*/*E* : 98.8:1.2)

### Example 7. (Z)-[11-(3-dimethylamino-propipyliden)-6,11-dihydro-dibenzo[b,e]oxepin-2-yl]-acetic acid hydrochloride

Active carbon (Norit SX Ultra) was added to a stirring solution of 6g (17.78mmol) of (Z)-[11-(3-dimethylamino-propyliden)-6,11-dihydro-dibenzo[*b*,*e*]oxepin-2-yl]acetic acid in tetrahydrofurane (282mL) at room temperature. The slurry was filtered, washed with tetrahydrofurane, HCl 12N was added until pH 0-1 and the mixture was heated at reflux temperature of the solvent for 15-20min. After that, the reaction mixture was cooled (15-20°C) and stirred for 30min, centrifuged, washed at first time in tetrahydrofurane (30mL) and followed by acetone, the obtained product was dried at 55-60 °C to obtain 6.20g of (Z)-[11-(3-dimethylamino-propyliden)-6,11-dihydro-dibenzo[*b*,*e*]oxepin-2-yl]acetic acid hydrochloride. (99.74% chromatographic purity; *Z*/*E*: 99.8:0.2).

A suspension of 5g (13.37mmol) of (Z)-[11-(3-dimethylamino-propyliden)-6,11-dihydro-dibenzo[*b*,*e*]oxepin-2-il]acetic acid hydrochloride in acetone (50mL) was heated at reflux temperature until the dissolution of Olopatadine hydrochloride, following this, acetone (250mL) was slowly added and was concentrated under reduced pressure distillation. The reaction mixture was cooled at 18-22 °C for 2h, centrifuged, washed with acetone, dried under vacuum to obtain 4.3g (Z)-[11-(3-dimethylamino-propyliden)-6,11-dihydro-dibenzo[*b*,*e*]oxepin-2-yl]acetic acid hydrochloride. (99.98% chromatographic purity; Z/ E: 99.99:0.01).

## Claims

1. A process for the preparation of (Z)-[11-3-(dimethylamino)propylidene]-6,11-dihydro-dibenz[b,e]oxepin-2-yl acetic acid hydrochloride of formula (1), 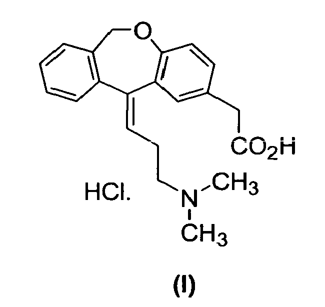 which comprises the reaction of compound of formula (III) 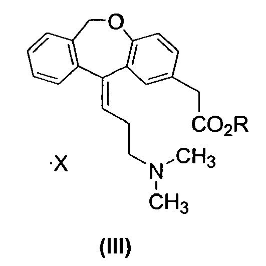 with a base, wherein R is C1-4 alkyl and X is an acid addition salt, to obtain compound of formula (II), 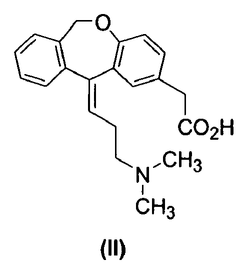 and reacting compound of formula (II) with hydrochloric acid, to obtain compound of formula (I), crystallizing with tetrahydrofurane and crystallizing with a ketone and water.

2. A process according to claim 1, wherein the base is sodium hydroxide.

3. A process according to claim 2 or 3, wherein R is methyl.

4. A process according to anyone of claims 1 to 3, wherein X is selected from hydrochloric acid, hydrobromide acid, succinic acid, oxalic acid, fumaric acid, maleic acid or sulfuric acid.

5. A process according to claim 4, wherein X is oxalic acid.

6. A process according to anyone of claims 1 to 5, wherein ketone is selected from acetone, methylisobutylketone, methylethylketone, diisobutylketone or mixture thereof.

7. A process according to claim 6, wherein ketone is acetone.

8. A process according to anyone of claims 1 to 7, which comprising the preparation of compound of formula (III) reacting compound of formula (IV), 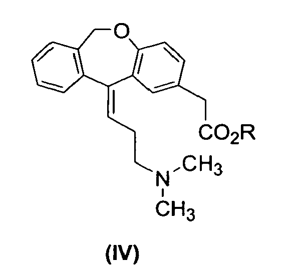 with an acid in one or more solvents or mixtures thereof, wherein R is C1-4 alkyl and X is as defined above.

9. A process according to claim 8, wherein acid comprises hydrochloric acid, hydrobromic acid, succinic acid, oxalic acid, fumaric acid, maleic acid or sulfuric acid.

10. A process according to claim 9, wherein acid is oxalic acid.

11. A process according to claim 10, wherein acid is oxalic acid dihydrate.

12. A process according to anyone of claims 8 to 11, wherein the one or more solvents and mixtures thereof are selected from one or more alcohols, one or more esters or mixtures thereof.

13. A process according to claim 12, wherein the one or more solvents are ethanol, ethyl acetate or mixtures thereof.

14. A process according to anyone of claims 8 to 13, wherein R is methyl.

15. A process according to anyone of claims 8 to 14, which comprises the preparation of compound of formula (IV) reacting compound of formula (V), 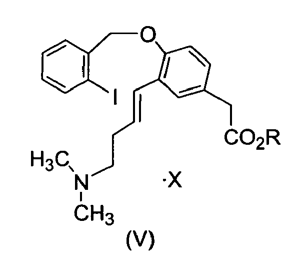 with a palladium catalyst, wherein R is C1-4 alkyl and X is as defined above.

16. A process according to claim 15, wherein R is methyl.

17. A process according to claim 15 or 16, wherein palladium catalyst is a fibre-anchored palladium homogeneous catalysts.

18. A process according to claim 17, wherein palladium catalyst is Fibrecat 1001® (Johnson Matthey).

19. A process according to anyone of claims 15 to 18, which comprises the preparation of compound of formula (V) reacting compound (VI), 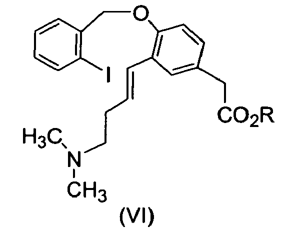 with an acid in one or more solvents or mixtures thereof, wherein R is C1-4 alkyl and X is as defined above.

20. A process according to claim 19, wherein R is methyl.

21. A process according to claim 19 or 20, wherein acid comprises hydrochloric acid, hydrobromic acid, succinic acid, oxalic acid, fumaric acid, maleic acid or sulfuric acid.

22. A process according to claim 21, wherein acid is oxalic acid.

23. A process according to claim 22, wherein acid is oxalic dihydrate.

24. A process according to anyone of claims 19 to 23, wherein the one or more solvents or mixtures thereof are selected from one or more alcohols, one or more esters or mixtures thereof.

25. A process according to claim 24, wherein the one or more solvents are ethanol, ethyl acetate or mixtures thereof.

26. A compound of formula (V), 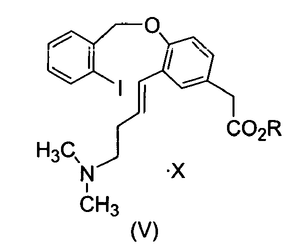 wherein R is C1-4 alkyl and X is an acid addition salt

27. A compound of formula (V) according to claim 26, wherein R is methyl.

28. A compound of formula (V) according to claim 26 or 27, wherein X is selected from hydrochloric acid, hydrobromide acid, succinic acid, oxalic acid, fumaric acid, maleic acid or sulfuric acid.

29. A compound of formula (V) according to claim 28, wherein X is oxalic acid.

30. A compound of formula (V) according to claim 26, (Z)-{4-[2-(4-dimethylamino-but-1-enyl)-benzyloxy]-3-iodo-phenyl}-acetic acid methyl ester oxalate.

31. A process for the preparation of compound of formula (V), 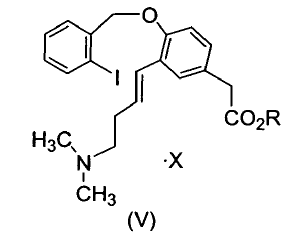 which comprises reacting compound (VI), 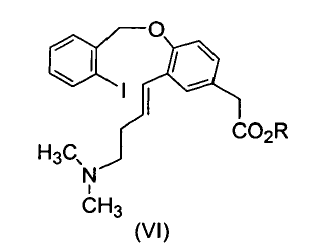 with an acid in one or more solvents or mixtures thereof, wherein R is C1-4 alkyl and X is an acid addition salt.

32. A process according to claim 31, wherein R is methyl.

33. A process according to claim 31 or 32, wherein acid comprises hydrochloric acid, hydrobromide acid, succinic acid, oxalic acid, fumaric acid, maleic acid or sulfuric acid.

34. A process according to claim 33, wherein acid is oxalic acid.

35. A process according to claim 34, wherein acid is oxalic acid dihydrate.

36. A process according to claims 31 to 35, wherein X is selected from hydrochloric acid, hydrobromide acid, succinic acid, oxalic acid, fumaric acid, maleic acid or sulfuric acid.

37. A process according to claim 36, wherein X oxalic acid.

38. A process according to anyone of claims 31 to 37, wherein solvents comprise the one or more solvents or mixtures thereof are selected from one or more alcohols, one or more esters or mixtures thereof.

39. A process according to claim 38, wherein the one or more solvents are ethanol, ethyl acetate or mixtures thereof.

40. A compound of formula (III), 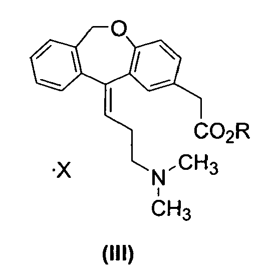 wherein R is C1-4 alkyl and X is an acid addition salt.

41. A compound of formula (III) according to claim 40, wherein R is methyl.

42. A compound of formula (III) according to claim 40 or 41, wherein X is selected from hydrochloric acid, hydrobromide acid, succinic acid, oxalic acid, fumaric acid, maleic acid or sulfuric acid.

43. A compound of formula (III) according to claim 42, wherein X is oxalic acid.

44. A compound of formula (III) according to claim 40, (Z)-[11-(3-dimethylamino-propilidene)-6,11-dihydro-dibenzo[*b*,*e*]oxepin-2-yl]acetic acid methyl ester oxalate.

45. A process for the preparation of compound of formula III, 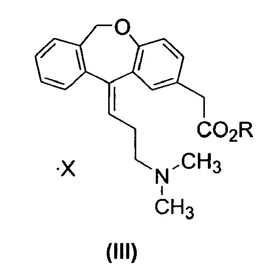 which comprises reacting compound (IV), 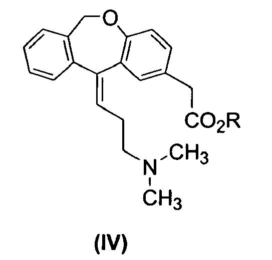 with an acid in one or more solvents and mixtures thereof, wherein R is C1-4 alkyl and X is an acid addition salt.

46. A process according to claim 45, wherein R is methyl.

47. A process according to claim 45 or 46, wherein acid comprises hydrochloric acid, hydrobromic acid, succinic acid, oxalic acid, fumaric acid, maleic acid or sulfuric acid.

48. A process according to claim 47, wherein acid is oxalic acid.

49. A process according to claim 48, wherein acid is oxalic acid dihydrate.

50. A process according to anyone of claims 45 to 49, wherein X is selected from hydrochloric acid, hydrobromic acid, succinic acid, oxalic acid, fumaric acid, maleic acid or sulfuric acid.

51. A process according to claim 50, wherein X is oxalic acid.

52. A process according to anyone of claims 45 to 51, wherein solvents comprise one or more solvents or mixtures thereof are selected from one or more alcohols, one or more esters or mixtures thereof.

53. A process according to claim 52, wherein the one or more solvents are ethanol, ethyl acetate or mixtures thereof.
